# EUROPEAN PATENT APPLICATION

(11) **EP 1 686 376 A1**
(43) Date of publication of application: **02.08.2006**
(21) Application number: 04025113.4
(22) Date of filing: 22.10.2004
(51) Int. Cl.: G01N 33/18

(54) **Automatable method for determining assimilable organic carbon and assessing microbial growth inhibition in water**

(71) Applicant: Eidg. Anstalt für Wasserversorgung, Abwasserreinigung und Gewässerschutz, 8600 Dübendorf (CH)
(72) Inventor: Egli, Thomas, 8706 Feldmeilen (CH); Hammes, Frederik, 8600 Dübendorf (CH)
(74) Representative: Blum, Rudolf Emil

(57) **Abstract**

A method for assessing assimilable organic carbon (AOC) levels and/or investigating the AOC-inoculum-relation and/or optimizing the method parameters, and/or for determining the microbial growth inhibition ability of a substance, said method comprising the steps of
a) providing a water sample having known or unknown AOC level,
b) distributing it into at least one set of at least triplicate vials, each set being intended for generating one data point,
c) inoculating each vial,
d) incubating the sets of inoculated vials, each set at a chosen incubation temperature,
e) sampling and determining the growth of the inoculated cells in each vial after at least one incubation time, said determination of the growth of the microbial cells being performed by
   (i) fluorescence-staining of the microbial cells,
   (ii) enumerating the stained cells by means of a flow cytometer,
f) generating an AOC-dependent and/or inoculum-dependent information is described.

Some advantages of the method are that it can be automated, and that AOC levels can be obtained at stationary phase or at pre-stationary phase.

## Description

The present invention concerns a method for the determination of microbiologically assimilable organic carbon (AOC) in water. The present method in particular concerns a faster, more realistic and more reliable method involving flow cytometry, and this method can easily be automated. The present invention has the additional advantage that the same principles can be used for the assessment of microbial growth inhibitory compounds in water.

Assimilable organic carbon (AOC) is a collective term describing the fraction of labile dissolved organic carbon (DOC) that is readily consumed by microorganisms, resulting in a proliferation of the organisms and a general increase in biomass. Hence, AOC is a major parameter concerning biological stability of water. Assessment of biological water quality and stability is very important, in particular in drinking water production and distribution. Regrowth of microorganisms and the formation of biofilms in drinking water production and distribution systems have already been shown to be statistically related to the concentration of AOC in the water (Van der Kooij, D. 2002. *Assimilable organic carbon (AOC) in treated water: determination and significance.* Encyclopedia of Environmental Microbiology. G. Bitton (Ed.). New York, NY, John Wiley, ISBN 0-471-35450-3; Escobar, I.C., Randall, A.A. and Taylor, J.S. 2001, *Bacterial growth in distribution systems: effect of assimilable organic carbon and biodegradable dissolved organic carbon,* Environmental Science and Technology, 35: 3442 - 3447.).

The conventional AOC test is a bioassay originally developed by Van der Kooij et al. in 1982 (see Van der Kooij, D., Visser, A. and Hijnen, W.A.M. (1982), *Determining the concentration of easily assimilable organic carbon in drinking water,* Journal of the American Water Works Association, 74: 540), and of which precise details can be found in Standard Methods (Greenberg, A. E., L. S. Clesceri, and A. D. Eaton (Ed.) 1993, *Standard methods for the examination of water and wastewater, 18th ed.,* American Public Health Association, Washington, D.C.). It is generally performed in that an AOC-free inoculum of (a) pure bacterial culture(s) is added to a pasteurized water sample in AOC-free vials, in that said inoculated water sample is incubated in batch cultivation mode until stationary phase is reached, in that the bacterial growth in the said water sample at stationary phase is quantified during three consecutive days end-point measurements, in that conventional plating on a general nutrient agar is used for enumeration of the growth (determination of colony forming units (cfu)), in that net-growth in the sample is taken as the numerical difference in cell concentration between the said water sample directly after inoculation and the average of the three stationary phase measurements, in that said net-growth is converted to acetate-carbon or oxalate-carbon equivalents by means of conversion values, and in that said conversion values are derived from calibration batch growth curves for the yield of said pure culture(s) on pure acetate or oxalate under similar incubation and inoculation conditions.

Hitherto, the preferred inoculum pure cultures were Pseudomonas fluorescence strain P-17 (ATCC 49642) (below referred to only as P-17) and Spirillum strain NOX (ATCC 49643) (below referred to only as NOX). Moreover, it is preferred that both pure cultures are used in the assay (either simultaneously or in parallel assays) since they target different types of AOC compounds. Other pure cultures and combinations of pure cultures have also been proposed. The prescribed inoculum density for said pure cultures is 500 cfu.mL⁻¹ final concentration, and the prescribed incubation temperature is 15 °C. End-point measurements are taken on day 7, 8 and 9 after inoculation. Plating enumeration is typically performed on R₂A-agar, requiring a further three to five days incubation at room temperature before the cfu can be quantified (Greenberg et al., 1993).

Evident shortcomings of the conventional method are the long incubation time (9 days), which is further lengthened by the incubation time of the plates (additionally 3 - 5 days), the tedious nature of plating as a quantification method, and the obvious question as to whether pure cultures with limited nutritional capabilities are able to realistically reflect the actual AOC content of a sample.

In Le Chevallier et al. (1993), (see Le Chevallier, M.W., Shaw, N.E., Kaplan, L.A. and Bott, *T.L.,"Development of a Rapid Assimilable Organic Carbon Method for Water",* Applied and Environmental Microbiology, 29(5): 1526-1531), and Kaplan et al. (1993) (see Kaplan, L.A., Bott, T.L. and Reasoner, D.J. (1993), *Evaluation and simplification of the assimilable organic carbon nutrient bioassay for bacterial growth in drinking water,* Applied and Environmental Microbiology, 59(5): 1532 - 1539) respectively, means for accelerating the standard method (Greenberg et al., 1993) are discussed such as an increased inoculum density (up to 1 × 10⁴ cfu.mL⁻¹ final concentration) and/or a higher incubation temperature (specifically 22 °C for said pure cultures). Said adaptations reduced the incubation time required to achieve stationary phase to approximately 5 days. As an alternative to plating, measuring the ATP content of cells by means of a luciferin-luciferase assay was proposed. Although this method removes the said additional incubation time for plating, this analysis has the disadvantages that it is expensive, requires a considerable degree of expertise to perform and interpret the data, and is prone to possible error of interpretation as bacteria can differ in their ATP content. The potential problem posed by the use of a limited number of pure cultures for the assay has not been addressed in said above adaptations.

Thus, there is still a need for - and it is the goal of the present invention - to provide a faster, more reliable, and easy to perform method for the assessment of AOC, which - as a further benefit - can be automated.

This goal has been achieved by providing a method and AOC apparatus as defined in the claims, e.g. by a method of the present invention comprising at least the steps of
a) providing at least one water sample,
b) distributing the at least one water sample into at least triplicate vials wherein said distribution is performed by means of sterile filtration, e.g. by using a syringe and filter,
c) adding a chosen amount of an AOC-free inoculum of microbial cells per vial,
d) incubating the at least triplicate inoculated vials of step c) at a chosen incubation temperature,
e) sampling to get an enumeration sample and determining the growth of the inoculated cells in each vial after at least one incubation time, whereby said determination of the growth of the microbial cells is performed by
   (i) fluorescence-staining of the microbial cells,
   (ii)enumerating the stained cells by means of a flow cytometer,
f) converting the inoculum net-growth (usually expressed as cells.mL⁻¹) into AOC (usually expressed as ug.L⁻¹ carbon),
in particular an automatable method, wherein the water sample is inoculated with an inoculum of microbial cells until the stationary phase is reached.

The cell counts can be converted to AOC using conversion values determined by performing the method of the present invention with water of known AOC concentration. Alternatively, a theoretical standard conversion value can be applied: Van der Kooij (2002) (reference above) suggested a rule of thumb value of 1 x 10⁶ bacterial cells per µg AOC.

Since any AOC present in the containers, syringes, pipettes, filters etc. used will also be determined and since such AOC might be different from that of the water sample and thus might be assimilated by the inoculum cells at a different rate, it is much preferred that all containers and manipulating means such as those mentioned above are AOC-free or non-AOC-releasing, in particular all containers and manipulating means used in steps a) to d). For confirming the quality of the containers and manipulating means, or if some impurities are expected, control samples with highly pure water should be simultaneously processed.

Suitable AOC-free containers and/or manipulating means can be purchased or prepared by known methods (see Greenberg et al. (1993) for detailed description on the requirements and preparation of containers and manipulating means). For said "distribution" step b) above, non-AOC-releasing filters should be used (e.g. 0.22 µm, Millex® -GP, Millipore). This step is essential to the inventive method as it removes particles that could interfere with the flow cytometry quantification step.

In general, the staining is performed by adding the commercially available staining agent directly to the sample in an amount as prescribed by the supplier. In order to assure that all cells are properly stained, the stained sample is again incubated in the dark for about 10 - 15 min. at about 25 °C (again determined by the specific stain that is used, and the prescriptions of the supplier).

A much preferred method is an automatable method, wherein the water sample is inoculated with a high density (up to 5 x 10⁴ cells.mL⁻¹ final concentration) natural microbial consortium and incubated at 30°C until stationary phase is reached. The automation will allow the measurement of complete growth curves as opposed to stationary phase end-point data, thus providing more representative data including insight into the growth kinetics.

In view of the hitherto preferred equipment, each water sample in step b) is distributed into vials of about 40 to 45 ml volume, such that each vial is filled with preferably the same amount of the filtered sample, in general about 40 ml.

After the desired incubation time, at least one enumeration sample is withdrawn from the incubated sample, in general 1 ml, and transferred to a new container, e.g., an Eppendorf tube, and then stained. Staining should be performed directly on the sample, with any required dilution steps done afterwards.

Depending on the type of flow cytometer and the water sample, a diluted sample would probably be used for enumeration. However, if the diluted sample is below a detectable limit, the undiluted sample is also measured.

In general, inoculation temperatures between 20 and 35°C are preferred, in particular temperatures between 25 and 35°C, especially temperatures around 30±2°C, most preferred temperatures of about 30°C.

A preferred inoculum density at the start is up to 5 x 10⁴ cells.mL⁻¹, usually between 5 x 10³ and 5 x 10⁴ cells.mL⁻¹, although lower inoculum densities work as well. As LeChevallier et al. (1993) have shown, a higher inoculum density shortens the time required to reach stationary phase. However, a too high inoculum concentration would reduce the sensitivity of the assay.

Contrary to the conventional AOC method described above, the method of the present invention uses fluorescent staining and flow cytometry for growth enumeration instead of conventional plating or ATP analyses. Fluorescent staining and flow cytometry has already been used for the quantification of microorganisms in water samples (see Veal, D.A., Deere, D., Ferrari, B., Piper, J. and Attfield, P.V., 2000, *"Fluorescence-staining and flow cytometry for monitoring microbial cells",* J. of Immunological Methods, 243:191-210). However, nobody has so far proposed to use flow cytometry in the determination of AOC levels, let alone suggested the specific steps and/or the great advantages of the inventive methods. The main advantages of the flow cytometry method of the present invention are:
- Due to the use of flow cytometry instead of the plating method, the final result of the measurement is obtained within 15 minutes compared to 3 - 5 days additional incubation needed for the plating method.
- The data generated by flow cytometry is statistically more reliable than the conventional plating method. For example, during plate counting, between 30 - 300 events are analysed per plate, and serial dilutions are used to obtain this range (e.g. 3 x 10⁵ cfu.mL⁻¹ are determined with at least three ten-fold dilutions). Flow cytometry analyses up to 60,000 events per 200 µl sample (e.g. 3 x 10⁵ cells.mL⁻¹ are determined with a single direct count), whereby the actual number of events that can be analysed is dependent on the flow cytometer used.
- In view of the statistically more reliable data, in general measurement at 1 incubation time at stationary phase is sufficient.
- Flow cytometry is much easier to perform than the ATP analysis method, and provides specific data on microbial growth (cells.mL⁻¹) opposed to estimations based on ATP-content.
- Much more samples can be processed rapidly with the flow cytometry method than with any of the other methods, providing the additional possibility of measuring complete growth curves and thus getting information on growth kinetics instead of only end-points.
- The automation possibilities of flow cytometry underscores all the points mentioned above. Suitable automation means are already developed in the relevant industry, however, they have never been combined in such a way that the method of the present invention might be performed without adaptation.
- Most importantly, flow cytometry is not restricted to the activity or culturability of the bacteria. This means that the flow cytometry method will quantify virtually all the bacteria that have grown in the sample, and not only the ones which are active or culturable at the point of analysis. Thus, this method does not only provide more reliable data for known culturable bacterial strains, but also enables the use of non-culturable cells.

Since the inventive method enumerates all cells present and is not bound to bacteria that are culturable, also a natural microbial consortium can be used as inoculum instead of the hitherto used selected pure cultures. This means that a much more realistic result can be generated. A natural microbial consortium has a broader nutritional range and is more adapted to use naturally occurring AOC compounds than a pure culture laboratory strain. This gives a more realistic estimate of the AOC content of a water sample. Hence, for a specific water, its indigenous microbial flora can be used for inoculation in AOC assays. However, if it is preferred for comparative reasons, the inventive method also allows the use of the standard pure culture strains (P-17 and NOX), either separately or in combination, as well as other pure cultures or composite mixtures of pure cultures.

One way of preparing said natural microbial consortium is to take a water sample, preferably from the source to be tested, and preferably using AOC-free, non-AOC-releasing means and containers, to filter this sample (usually about 40 ml) with a non-AOC-releasing microfilter (0.22 µm), into AOC-free vials (volume in general about 40 to 45 ml), to inoculate said filtered sample with about 100 µl of the original unfiltered water, to incubate said inoculated samples at a preferred temperature (e.g. 30 °C) until stationary phase is reached, and to measure stationary phase (thus growth) by means of fluorescent staining and flow cytometry. Such inoculum can be stored, e.g. at about 5 °C for a typical period of about 6 months.

In a preferred embodiment of the present invention, all bacteria are stained before flow cytometry using a general DNA-targeting dye (e.g. SYBR® Green (Molecular Probes) or SYTO9 (Molecular Probes)). However, the present invention also allows the use of fluorescent in situ hybridisation (FISH) probes, specific antibodies, or viability stains in order to acquire more complex information on the composition and physiological state of the consortium in the AOC assay.

In one aspect of the inventive method, the incubation is performed until stationary phase is reached.

A preferred embodiment of said method for assessing AOC levels in water is characterized by the steps of
a) providing at least one water sample,
b) distributing the at least one water sample into at least triplicate AOC-free vials, wherein said distribution is performed by means of sterile filtration in general using AOC-free, non-AOC-releasing syringe and filter,
c) adding a chosen amount per vial (usually between 5 x ¹⁰³ - 5 x 10⁴ cells.mL⁻¹ final concentration) of an inoculum of cells of preferably a natural microbial consortium,
d) incubating the at least triplicate inoculated vials of step c) at a temperature of usually 20°C to 35°C, preferably 25 to 35°C, in particular at about 30±2 °C, especially at about 30 °C, until stationary phase is reached,
e) sampling to get an enumeration sample and determining the growth of the inoculated cells in the sample, whereby said determination of the growth of the inoculated cells is performed by
   (i) fluorescence-staining of the microbial cells,
   (ii)enumerating the stained cells by means of a flow cytometer,
f) converting the inoculum growth into AOC, whereby at least steps a) to d) are performed using AOC-free and non-AOC-releasing containers, in particular vials, and AOC-free and non-AOC-releasing manipulating means, in particular syringes and filters.

In view of the more reliable measurements less data points (data point refers to the average of the measurements of incubated samples taken from at least three vials of the same water sample incubated at one specific incubation temperature for one specific incubation time) are needed than with plating. In particular if it is known or expected from experience where stationary phase begins, in said stationary phase one data point has proved to usually be sufficient, although more data points are preferred in view of reliability.

With a specific embodiment of the method of the present invention, it is also possible to determine the best incubation temperature for fast AOC consumption, i.e. growth. Said best incubation temperature for a specific pure culture or a specific natural microbial consortium can be determined with a method comprising the steps of
a) preparing at least one water sample spiked with at least one known AOC compound or, preferably, one natural AOC sample
b) distributing said sample into at least 2 sets of at least triplicate vials, wherein said distribution is performed by means of sterile filtration, preferably using AOC-free, non-AOC-releasing syringe and filter
c) adding a chosen amount (usually up to 5 x 10⁴ cells.mL⁻¹ final concentration) of the chosen inoculum of microbial cells of interest per vial
d) incubating said at least 2 sets of at least triplicate inoculated vials of step c) each set at one of at least two chosen temperatures
e) determining the growth of the inoculated cells in each vial at minimum 4, preferably at minimum 5, time points whereby said determination of the growth of the microbial cells is performed by
   (i) fluorescence-staining of the microbial cells,
   (ii) enumerating the stained cells by means of a flow cytometer,
f) determining the best of the investigated temperatures and stationary phase time and/or exponential growth time, and optionally redoing steps a) to d) at temperatures close to the best temperature found in the previous run,
at least steps a) to d) being performed using AOC-free and non-AOC-releasing containers, in particular vials, and AOC-free and non-AOC-releasing manipulating means, in particular syringes and filters.

Usually the first temperatures investigated, in particular for the natural microbial consortium, is in the range of 20 to 35 °C, preferably 25 to 35°C, in particular 30 ± 2 °C.

Due to the fact that flow cytometry analysis is quick, easy to perform, relatively cheap, and that it can be automated, the inventive method can also be used as a tool for acquiring preliminary information on the AOC level of a water sample before stationary phase is actually reached.

It has namely been found that the specific growth rate (µ) of a natural microbial consortium at an incubation temperature of 30 ± 2 °C is exponential between at least 8 and 20 h if an inoculum density of approximately 1 x 10⁴ cells.mL⁻¹ final concentration is used, and that this specific growth rate is related to the initial AOC concentration in the water. Thus, by sampling at at least 3, preferably 5 different times within this period (8 - 20 h), enumerating the cells with fluorescent staining and flow cytometry, determining the specific growth rate with the acquired data, and then comparing the specific growth rate to standard specific growth-rates, an estimation of the AOC concentration can be obtained within less than 20 h after inoculation. This is a very important finding since in case of accidental pollution of a drinking water supply, rapid information on the AOC concentration is required. Thus, it is also an aspect of the present invention to provide a method for assessing AOC levels in a water sample based on growth kinetics. Said fast method comprises the steps of
a) providing at least one water sample,
b) distributing said sample into at least triplicate vials, wherein said distribution is performed by means of sterile filtration, preferably using syringe and filter,
c) adding a chosen amount (usually up to 5 x 10⁴ cells.mL⁻¹, preferably about 1 x 10⁴ cells.mL⁻¹ final concentration) of an inoculum per vial,
d) incubating the at least triplicate inoculated vials of step c) at a chosen incubation temperature for a time period shorter than the minimum time to achieve stationary phase in the growth curve,
e) sampling to get an enumeration sample and determining the growth of the inoculated cells in each vial at minimum 4, preferably 5 different incubation times during (exponential) growth on the batch growth curve,
   whereby said determination of the growth of the microbial cells is performed by
   (i) fluorescence-staining of the microbial cells,
   (ii) enumerating the stained cells by means of a flow cytometer,
f) determining the specific growth rate in the sample using the data acquired in step e)
g) comparing the specific growth rate to standard specific growth rates. As already mentioned above, also in this method it is very important that in particular all means and containers used in steps a) to d) are AOC-free and non-AOC-releasing.

While with different natural inoculums investigated so far generally exponential growth was found, this method is also applicable to non exponential growth, provided that sufficient data points are measured to get a clear growth curve. However, this method is preferably applied in the case of at least almost exponential growth.

Said standard specific growth rates can be determined by a method that follows the steps outlined above except that in step a) several, in general at least 5, water samples are prepared by spiking samples of water of known purity, in general very pure water, each with a specific, different, preferably pre-calculated AOC concentration, said at least 5 AOC concentrations being within the range to be generally assayed. The AOC used can be either a pure AOC compound (e.g. acetate) or a mixture of known AOC compounds. The optimal incubation temperature for this method can be determined with the method outlined above.

For the kinetic-based (exponential growth) method, an inoculum density of about 1 x 10⁴ cells.mL⁻¹ is preferred.

With both methods of the present invention, i.e. the growth kinetic-based method and the more accurate stationary phase method, the overall reduced time required for getting the result is a great advantage of the inventive method compared to the hitherto used method. In a preferred embodiment of the present invention, stationary phase is usually reached within 24 - 48 h after inoculation. An analysis of 20 different samples showed that after 24 h, the growth measured, or the AOC determined, respectively, corresponds to 89 ± 17 % of the result at stationary phase, and that at 40 - 48h the result corresponds to 96.9 ± 0.6 % of the result at stationary phase.

A further application of the present invention is the analysis of the microbial growth inhibitory effect of specific chemical compounds, or unidentified compounds in a given water sample. This is possible because the proposed AOC bioassay is based on microbial growth. Analysis of growth inhibition is based on both the final cell yield and the growth kinetics.

When the growth inhibition caused by a particular compound is analysed, the test is carried out by adding the compound at different concentrations to water samples known to support growth. Such water samples can be natural (e.g., river water) or synthetic (water with a known AOC mixture added). Growth of the samples spiked with the compound is then compared to growth in the water without any compound addition.

When the growth inhibition caused by unknown compounds in a given water sample is analysed, the test is carried out by adding a known concentration of (a) known AOC compound(s) to the water sample. Said added AOC compound can be a single compound such as glucose or acetate, or a mixture of AOC compounds, as long as it is known and tested to support growth of the inoculated cells in clean water samples. Furthermore, it is preferred that a complete mineral solution (AOC free) is added to ensure that growth inhibition is a result of a toxic compound and not a lack of a required mineral such as nitrogen or phosphate. Growth of the samples spiked with the AOC and the minerals is then compared to growth of unmodified samples and also with growth in a clean water sample with the AOC and minerals added. Such clean water sample could for example be commercially available natural (not carbonated) bottled water.

Said growth inhibition assays allow the use of either a natural microbial consortium as inoculum, or a specifically selected pure culture. The natural microbial consortium is preferred when general growth inhibition is analysed, while a pure culture is preferred when specific compounds e.g., antibiotics, are analysed.

Growth inhibition caused by a specific compound can be analysed with a method specifically comprising the steps of
a) providing at least one water sample spiked with at least one known AOC compound at at least one chosen concentration,
b) adding to said water sample the specific compound to be tested in a chosen concentration
c) distributing said sample into multiple identical sets of at least triplicate AOC-free vials, wherein said distribution is performed by means of sterile filtration using a non-AOC-releasing syringe and filter
d) adding a chosen amount (up to 5 x 10⁴ counts.mL⁻¹ final concentration) of an inoculum per vial
e) incubating the at least triplicate inoculated vials of step d) at a selected temperature,
f) sampling to get an enumeration sample and determining the growth of the inoculated cells in each vial after different incubation times until stationary phase is reached and maintained for establishing at least one growth curve , wherein said determination of the growth of the microbial cells is performed by
   (i) fluorescence-staining of the microbial cells (ii)enumerating the stained cells by means of a flow cytometer.

Sufficient incubation times or time points, respectively, usually are at least 4 time points, in general 5 to 10 time points per growth curve. Preferably at least 3, more preferred about 5 to 8 growth curves are established, each with an other concentration of the substance to be tested.

When the growth inhibitory nature of an unknown water sample is to be analyzed, the same steps as outlined above are performed, except steps a) and b) as far as spiking with a known AOC compound or mixture of AOC compounds and adding the specific compound to be tested is concerned.

A suitable AOC apparatus for performing the method of the present invention, and which includes the automation already referred to above, comprises a first incubation chamber for incubating the at least triplicate vials of the at least one inoculated water sample described above. Said incubation chamber preferably is suitable for simultaneous incubation of multiple samples/vials and preferably allows selecting between multiple incubation temperatures. Said first incubation chamber is equipped with a first auto-sampling device for selecting and withdrawing an enumeration sample to be stained, and placing said enumeration sample in a suitable staining container. Care should be taken that the sampling is done in such a manner that the remaining sample is not contaminated with AOC and that all materials of the sampling device coming into contact with the sample are AOC-free and non-AOC-releasing. Said auto-sampling device may comprise or be connected to an automated stain addition means and optionally a diluting means, a means for transporting the stained sample to a stained sample incubation means, that may be an area located within the first incubation chamber or, preferably to a second incubator for incubating said stained sample. Said second incubator could be connected to an automated fetching means for fetching at least part of said stained sample from the incubation chamber and transporting it to the flow cytometer, a flow cytometer equipped with at least one fluorescence detector and preferably at least two fluorescence detectors for measuring at different wavelengths. Although with one detector acceptable results can be obtained, usually two detectors are preferred. More than two detectors for general analyses are not necessary, however, if for example selective staining is used to get additional information, three or more detectors might be advantageous. As already mentioned above, between the stained sample incubation means and the flow cytometer, a means for diluting the selected sample may preferably be provided in order to ensure that the cell number is within a measurable range. From sampling at the desired incubation time, to the flow cytometry analysis, all steps can be fully automated including the conversion of the flow cytometry results to AOC, using appropriate software. In a preferred embodiment one auto-sampler is used that includes all means needed for taking at least one sample, staining said sample, optionally diluting said sample, and transporting said stained sample to the flow-cytometer. Several technological designs, which could suffice for this purpose with various degrees of adaptation, already exists in the industry, e.g., a flow injection apparatus available from FIAlab Instruments, Inc., Seattle, USA under the name FIAlab®, or an auto-sampler robot available from Partec GmbH, Münster, Germany and sold as "Robby® ".

Although such automated devices for the different steps outlined above already exist in the flow cytometry industry, these have up to now not been used for this purpose or in this specific configuration. Other embodiments are possible and will clearly surface to the skilled person reading this description.

Although the above apparatus has been described for automated analysis it has to be understood that also partial automation or no automation is encompassed by the present invention.

The invention will be better understood and objects other than those set forth above will become apparent when consideration is given to the following further description thereof, in particular the examples and the annexed drawings, wherein:
**Figure 1** is a comparison of the hitherto generally applied AOC bioassay with pure cultures and plating and the basic steps of the method of the present invention.
**Figure 2** shows typical examples of fluorescence histogram and dot-plot data and enumeration gates for P. *fluorescence* strain P-17 in pure water (A - C) and a natural microbial consortium in river water (D - F), after staining with SYBR® Green and flow cytometric analysis. Gates R1 on the combined fluorescence dot-plots (C and F) are used for enumeration purposes, allowing clear distinction between the background and the stained sample in natural water.
**Figure 3** shows a growth curve (cells.mL⁻¹ vs. the time in days) of identically prepared samples of P. *fluorescence* strain P-17 in acetate, one series measured by flow cytometry, the other one by plating (cfu.mL⁻¹) (see Example 1).
**Figure 4** shows growth curves (cells.mL⁻¹ vs. time in hours, enumerated by flow cytometry) of a natural microbial consortium on 100 µg.L⁻¹ acetate-carbon at 15°C and 30°C, respectively, for 9 days, wherein commercial mineral water was used as water matrix and served as controls (see Example 2a).
**Figure 5** shows detailed growth curves (cells.mL⁻¹ vs. time in hours (enumerated by flow cytometry)) of a natural microbial consortium in different concentrations of river water, whereby
**Figure 5a** shows the growth of a natural microbial consortium on 5 dilutions of river water during a 24 h period at 30 °C incubation,
**Figure 5b** shows specific growth-rates as determined with the time points 8 h to 16/19/24 hwhich represents a time period when growth was exponential in the samples. Respective specific growth rates are indicated on the graph. The AOC concentration in 100 % river water is about 60µg.L⁻¹.
**Figure 6a** illustrates the method of the present invention performed within an automated AOC apparatus wherein a second incubation chamber is used as stained sample incubation means, and
**Figure 6b** illustrates the method of the present invention performed within an automated AOC apparatus wherein a loop incubator is used as stained sample incubation means.

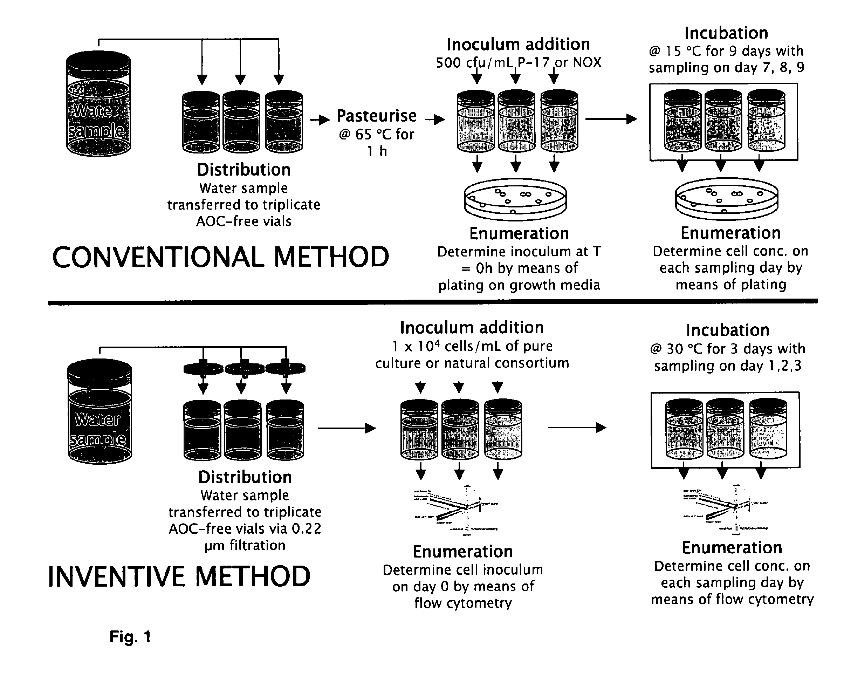

As already shortly addressed above, Figure 1 shows the hitherto generally applied AOC test with pure cultures and plating (Figure 1 top) and the method of the present invention (Figure 1 bottom). For both, water samples were split into triplicate partial samples or triplicate vials, respectively and determination of the inoculum at time T = 0 hours was performed. As can be seen, the inventive method does not include a pasteurization step. The purification step is sterile filtration, removing cells and cell parts or other larger solids. The sterile filtration instead of the pasteurization has the advantage that pasteurization can lead to the breakdown of complex carbon molecules, resulting in additional AOC formation.

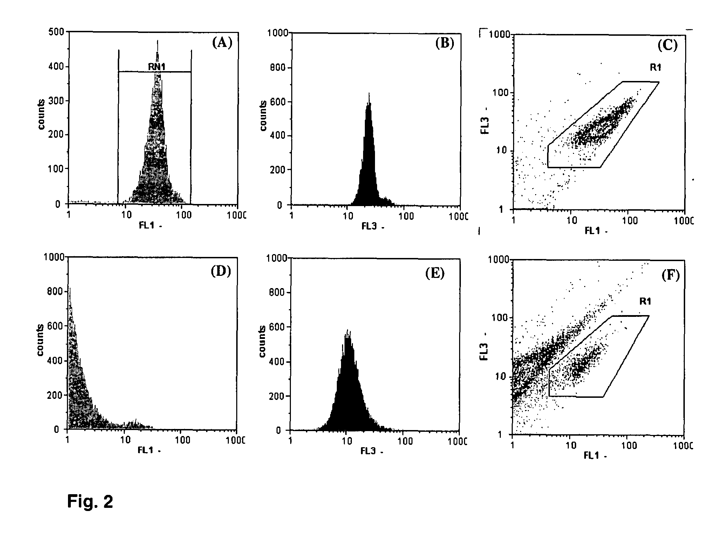

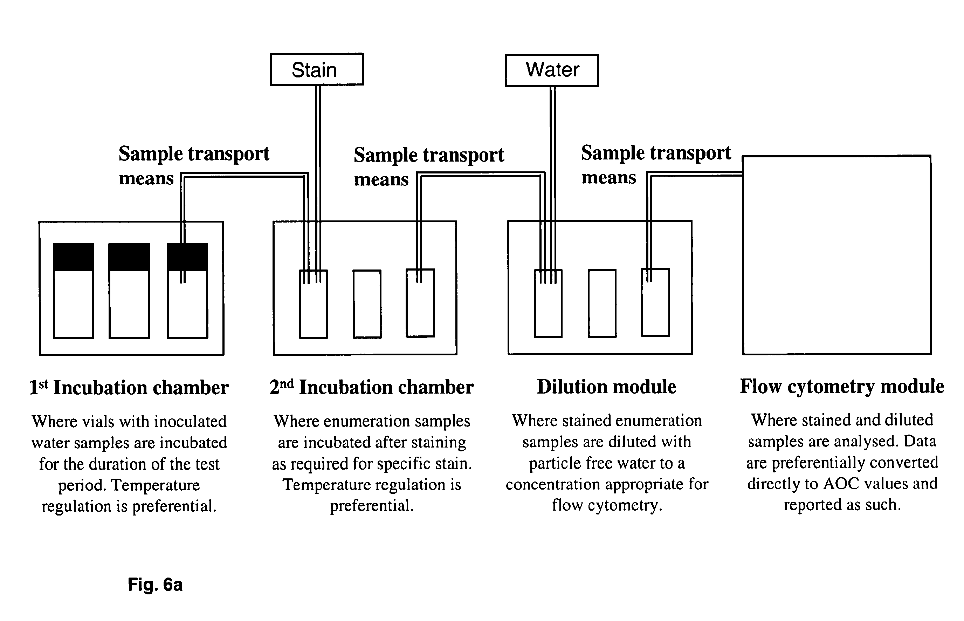

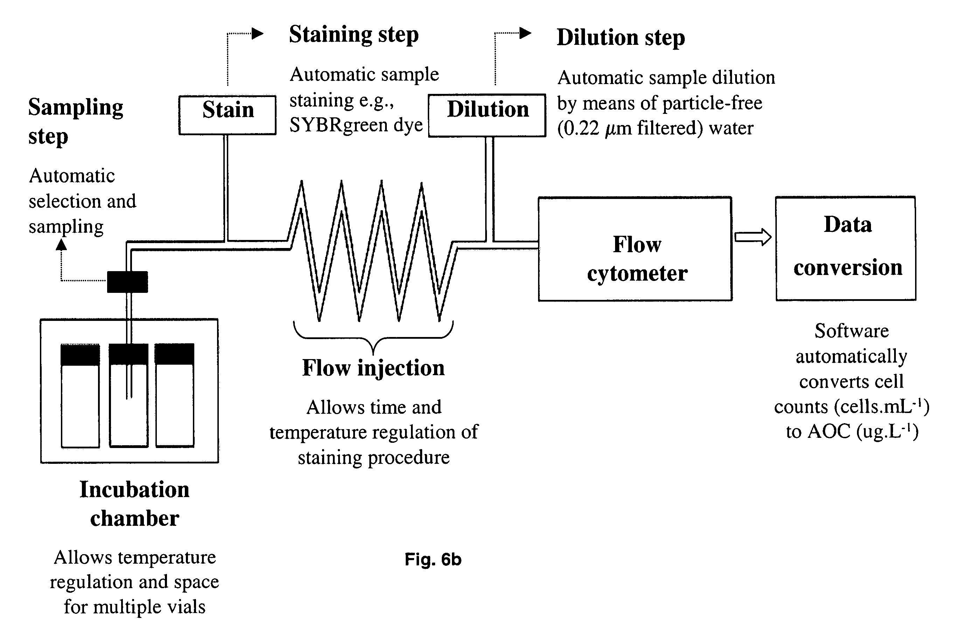

A method of the present invention with information on the flow cytometer comprising AOC apparatus is shown in Figure 6. Such apparatus may comprise a first incubation chamber for multiple samples, equipped with a first auto-sampling device for selecting (and withdrawing) a sample to be stained, a stain addition means, whereby said stain addition means encompasses an automatic stain dosing unit, an automatic sample dilution means, a means for fetching the stain comprising sample and transporting it to the flow cytometer, and a flow cytometer equipped with at least one, preferably two fluorescence detectors for detecting different wave lengths (see also Figure 2). In Figure 6, the device is generalized as far as the auto sampler, the addition means and the second incubation step are concerned. However, suitable embodiments surface to the skilled person from the description.

Figure 2 shows fluorescence histogram data and enumeration gates for *P. fluorescence* strain P-17 in pure water (A - C) and a natural microbial consortium in river water (D - F), after staining with SYBR® Green and flow cytometric analysis. The fields (A) and (D) are the results measured with one detector, the fields (B) and (E) with a second detector. As also shown in Figure 2, gates R1 on the combined fluorescence dot-plots (C and F) can be used for enumeration purposes, allowing clear distinction between the background and the stained sample in natural water.

While usually a non-selective staining, i.e. a staining by which all bacteria present in a sample are marked, is used, it is also possible to get further information by selective staining. Methods for selective staining are e.g. described in D.A. Veal et al., loc cit. Such selective staining allows to distinguish between viable and non-viable cells or between specific types of microorganisms.

As can be seen from the above description, the present invention provides a general method that is primarily intended for assessing AOC levels in water. However, the same method can also be applied to investigate the growth potential of cell cultures, to determine optimal process parameters etc., dependent on whether the sample used in step a) has unknown or known AOC level and dependent on the data generated in step f). The same method can also be used to assess the growth inhibitory nature of specific chemical compounds or of an unknown water sample, as described above. All methods of the present invention have common steps b) to e). Such a general method may be defined as a method for assessing AOC levels and/or investigating the AOC-inoculum-relation and/or optimizing the method parameters, said method comprising the steps of
a) providing at least one water sample, said sample having known or unknown AOC level,
b) distributing the at least one water sample into at least one set of at least triplicate vials wherein said distribution preferably is performed by means of sterile filtration, preferably using syringe and filter, each set being intended for generating one data point,
c) adding a chosen amount of an inoculum of microbial cells per vial,
d) incubating the at least one set of inoculated vials of step c) each set at a chosen incubation temperature,
e) sampling to get an enumeration sample and determining the growth of the inoculum in each vial after at least one incubation time, whereby said determination of the growth of the microbial cells is performed by
   (i) fluorescence-staining of the microbial cells,
   (ii) enumerating the stained cells by means of a flow cytometer,
f) generating an AOC and/or inoculum dependent information,
wherein at least steps a) to d) are performed using AOC-free and non-AOC-releasing containers, in particular vials, and AOC-free and non-AOC-releasing manipulating means, in particular syringes and filters.

Two possibilities for an automated AOC apparatus are schematically illustrated in Figures 6a and 6b. The apparatus in Figure 6a comprises a second incubation chamber for the incubation of the stained samples. In performing the inventive method in this apparatus, the water sample is first divided and filtered into multiple vials that are then inoculated and placed in a first incubation chamber. In the first incubation chamber, the vials with the inoculated water samples are incubated for the duration of the test period at the chosen incubation temperature. In the first incubation chamber, temperature regulation is preferred. Such temperature regulation allows to adapt the incubation temperature to a specific system and/or to perform temperature-dependent studies. After the desired incubation time, enumeration samples are taken and placed in new containers. Said new containers are placed in a second incubation chamber either prior to being filled with sample or after having been filled with the sample. In the second incubation chamber, the samples are then provided with stain. Although in the case of containers that are first filled with the enumeration sample and then placed in the incubator staining might be performed prior to placing the filled container in the incubator, staining in the incubator is preferred. It is even possible for better mixing to first add the stain and then the enumeration sample. After staining the enumeration samples, said samples are incubated in said second chamber as required for the specific stain. Also in the second incubation chamber it is preferred that the temperature can be regulated. After incubation, part of the stained sample is withdrawn from its container and transported to a new container where it is diluted. For said dilution, particle free water is used in an amount needed to get a concentration appropriate for flow cytometry. After dilution the diluted sample is transported to the flow cytometry module where the stained and diluted samples are analysed. The data are preferentially converted directly to AOC values and reported as such. All steps from taking the enumeration sample until the flow cytometry data conversion are preferably performed in a fully automated manner.

In the alternative embodiment of an AOC apparatus shown in Figure 6b, after the division of the water sample into multiple vials, inoculating them and incubating them in an incubation chamber as described above, an enumeration sample is withdrawn and continuously processed as described now. The first step, the sampling step comprises an automatic selection and sampling, followed by a staining step wherein in an automatic manner stain, e.g. SYBRgreen dye, is added. The stained sample proceeds into a flow injection apparatus (loop incubator) that allows time and temperature regulation of the staining procedure. After the desired residence time in the loop incubator/flow injection, the sample is automatically diluted by means of particle-free water and then forwarded to the flow cytometer for enumeration followed by data conversion, wherein a software automatically converts cell counts (cells.mL⁻¹) to AOC (µg.L⁻¹) . For the addition of stain and water for dilution, the sample comprising and transporting tube is connected to a stain dosing unit located on the incubation chamber side of the loop incubator and a dilution water dosing unit located between the loop incubator and the flow cytometer.

The invention is now further described by means of some non-limiting examples.

### Example 1: Application of flow cytometric (FCM) enumeration in the conventional AOC bioassay, as compared to enumeration by plating.

This Example shows the advantage of the present invention compared to the conventional AOC assay. Water samples have been incubated with *Pseudomonas* P-17 at 15°C incubation, and on the one hand FCM is used for enumeration, on the other hand plate counting.

The following experimental procedure was followed:

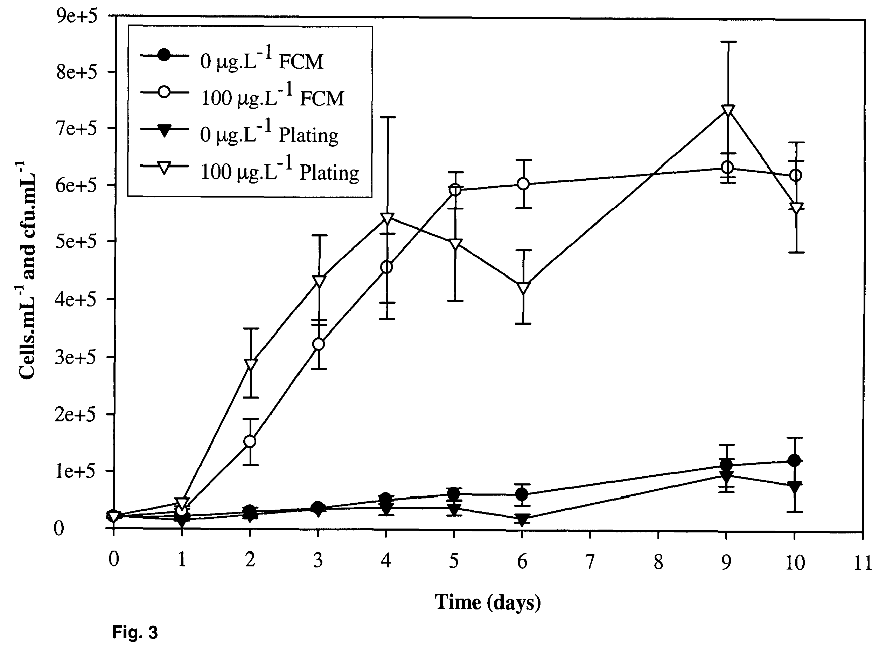

100 ug.L⁻¹ acetate-carbon were added to deionised water. The solution obtained was filter sterilised and then inoculated with *P. fluorescence* P-17 to a final concentration of about 1x10⁴ cells.mL⁻¹. The inoculated solution then was incubated at 15 °C for 10 days. Samples were taken at regular intervals and measured with flow cytometry (after SYBR® Green staining), and with plating on common R₂A agar. The results are shown in Figure 3, wherein all data points are average values for triplicate measurements with standard deviation indicated.

As can be seen from Figure 3, FCM detects a stationary phase from day 5 onwards, with no significant change up to day 10. Contrary thereto, the plating results show more variance and it is less evident where the stationary phase exactly starts and what number of counts corresponds to said phase.

The empirical yield value for P. *fluorescence* P-17 is 4.1 x 10⁶ cfu per µg acetate-carbon. The yield results above suggests a slightly higher value of 5.0 x 10⁶ counts per µg acetate-carbon presumably due to the enumeration of all cells.

This example shows that opposed to the plate count method FCM is easier to perform, much quicker, and statistically more reliable.

### Example 2: Application of flow cytometric (FCM) enumeration to an advanced AOC bioassay, in which a natural microbial consortium is used instead of a pure culture

### Example 2a: This example demonstrates the use of natural microbial consortium applied to water with known AOC level.

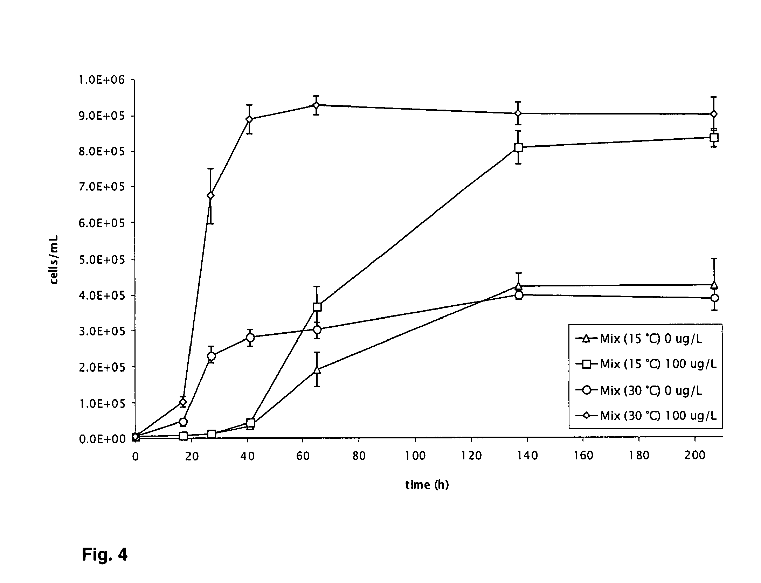

The experiment was performed in that 100 µg/L acetate-carbon were added to commercial mineral water. The resulting solution was filter sterilised and inoculated with a *natural microbial consortium* to a final concentration of about 1x10⁴ cells.mL⁻¹. These were then incubated at either 15 °C or 30 °C for 10 days. Samples were taken at regular intervals and measured with flow cytometry (after SYBR® Green staining). The results are shown in Figure 4, wherein all data points are average values for triplicate measurements with standard deviation indicated.

This example shows that such a consortium can be enumerated successfully with FCM and that such a consortium can be used to measure pure AOC compounds (e.g. acetate). This example also illustrates why a higher incubation temperature is preferred in the proposed method, as stationary phase is reached much quicker.

### Example 2b: This example demonstrates the use of natural microbial consortium applied to natural mixtures of AOC compounds (e.g. river water).

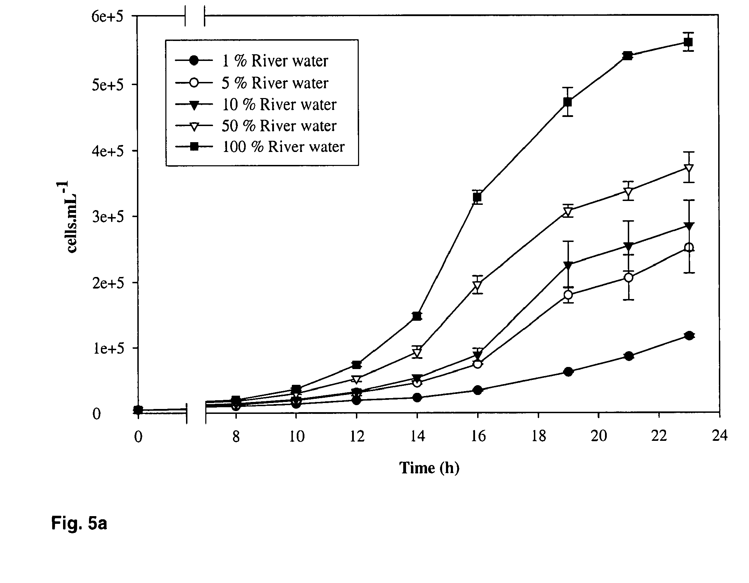

In this example, different dilutions of river water were filter sterilised and inoculated with a *natural microbial consortium* to a final concentration of about 1x10⁴ cells.mL⁻¹. These were then incubated at 30 °C for 42 hours. Samples were taken at regular intervals and measured with flow cytometry (after SYBR® Green staining). The results of the whole 42 h period are shown in Figure 5a, wherein all data points are average values for triplicate measurements with standard deviation indicated.

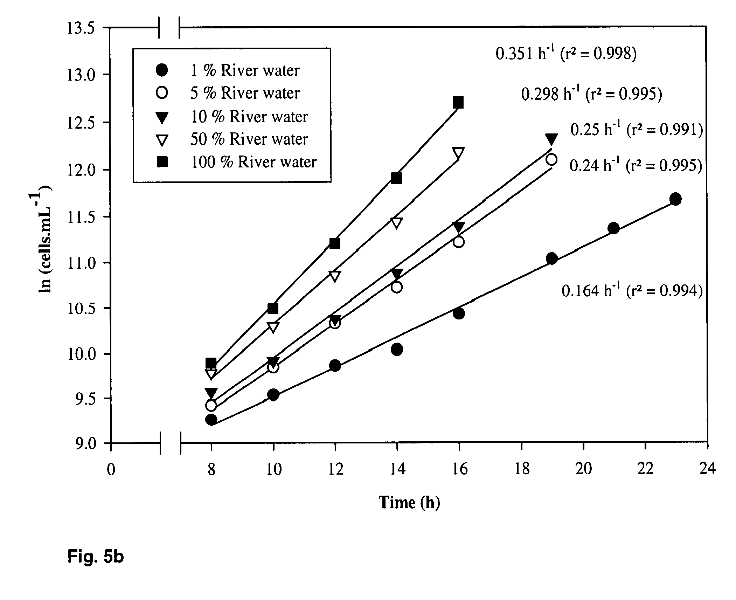

By plotting the natural logarithm (ln) of the cells.mL⁻¹ vs. the time in hours for the data points taken at 7 h, 15 h, 17 h, and 19 h, the exponential growth in an early stage of the incubation could be shown and the specific growth rate determined (see Figure 5b).

Example 2 shows that by the method of the present invention, natural microbial consortia (mixtures of natural microbial populations) and higher temperatures can be used, and that already after less than one day usable information on the AOC level of a water sample can be obtained.

Higher temperatures (like 30 °C) allow faster growth, thus faster obtaining a test result and natural consortia have a much broader nutritional range such that their use enables to detect a wider range of AOC than the use of one pure culture or makes the use of more than one pure culture superfluous. A natural microbial consortium can be enumerated successfully with FCM and used to measure natural mixtures of AOC compounds (e.g. river water).

Figures 5a and 5b show rapid growth response (between 8 - 23 hours) to the AOC, and exponential growth in the range of 14 to 22 hours, while the different concentrations of river water could clearly be detected.

## Claims

1. A method for assessing AOC levels and/or investigating the AOC-inoculum-relation and/or optimizing the method parameters, and/or for determining the microbial growth inhibition ability of a substance, said method comprising the steps of
a) providing at least one water sample, said sample having known or unknown AOC level,
b) distributing the at least one water sample into at least one set of at least triplicate vials wherein said distribution preferably is performed by means of sterile filtration, preferably using syringe and filter, each set being intended for generating one data point,
c) adding a chosen amount of an inoculum of microbial cells per vial,
d) incubating the at least one set of inoculated vials of step c) each set at a chosen incubation temperature,
e) sampling to get an enumeration sample and determining the growth of the inoculated cells in each vial after at least one incubation time, whereby said determination of the growth of the microbial cells is performed by
(i) fluorescence-staining of the microbial cells,
(ii) enumerating the stained cells by means of a flow cytometer,
f) generating an AOC-dependent and/or inoculum-dependent information,
wherein at least steps a) to d) are performed using AOC-free and non-AOC-releasing containers, in particular vials, and AOC-free and non-AOC-releasing manipulating means, in particular syringes and filters.

2. The method of claim 1 for assessing AOC levels in water comprising the steps of
a) providing at least one water sample,
b) distributing the at least one water sample into at least triplicate vials wherein said distribution is performed by sterile filtration,
c) adding a chosen amount of an inoculum of microbial cells per vial,
d) incubating the at least triplicate inoculated vials of step c) at a chosen incubation temperature,
e) sampling to get an enumeration sample and determining the growth of the inoculated cells in each vial after at least one incubation time, whereby said determination of the growth of the microbial cells is performed by
(i) fluorescence-staining of the microbial cells,
(ii) enumerating the stained cells by means of a flow cytometer,
f) converting the inoculum growth characteristic to AOC,
wherein at least steps a) to d) are performed using AOC-free and non-AOC-releasing containers, in particular vials, and AOC-free and non-AOC-releasing manipulating means, in particular syringes and filters.

3. The method of claim 1 or 2 wherein the predetermined incubation time in step e) is a time at which the inoculum has reached stationary phase, and wherein in step f) the cells enumerated by flow cytometry are directly converted to AOC.

4. The method of claim 3, wherein the incubation temperature is 20°C to 35°C, in particular about 30°C, and the incubation time is at least about 2 days, preferably about 2 to 3 days, whereby data points at minimum 1, preferably at 1 or 2 different incubation times are generated.

5. The method of claim 2 wherein the incubation time in step e) is a time that is shorter than the minimal time to get stationary phase, and wherein in step f) the inoculum growth characteristic is the specific growth rate and wherein the conversion is made in that the specific growth rate is compared to standard specific growth rates.

6. The method of claim 5, wherein each incubation time is in the range of 8 to 20 hours, whereby data points at minimum 4, preferably at 5 different incubation times within the specified range are generated.

7. The method of claim 5 or 6, wherein the standard growth rates at a specific incubation temperature are determined by a method comprising the steps of
a) preparing at least one water sample for each standard AOC level, each of said samples comprising a chosen AOC level in water,
b) distributing each sample into at least triplicate vials wherein said distribution preferably is performed by means of sterile filtration,
c) adding a chosen amount of an inoculum of microbial cells of interest per vial,
d) incubating each of the inoculated triplicate vials of step c) at the chosen incubation temperature,
e) sampling to get an enumeration sample and determining the growth of the inoculated cells in each vial after at least three different incubation times that are shorter than the minimal time to get stationary phase, wherein said determination of the growth of the microbial cells is performed by
(i) fluorescence-staining of the microbial cells,
(ii) enumerating the stained cells by means of a flow cytometer,
f) determining the specificgrowth rate in dependence of the chosen AOC level,
wherein at least steps a) to d) are performed using AOC-free and non-AOC-releasing containers, in particular vials, and AOC-free and non-AOC-releasing manipulating means, in particular syringes and filters.

8. The method of claim 1 for determining the optimum incubation temperature, in particular for use in the method of anyone of the preceding claims, comprising the steps of
a) preparing at least one water sample spiked with at least one known AOC compound or a natural AOC sample,
b) distributing said sample into at least 2 sets of at least triplicate vials wherein said distribution preferably is performed by means of sterile filtration,
c) adding a chosen amount of an inoculum of microbial cells of interest per vial,
d) incubating said at least 2 sets of at least triplicate inoculated vials of step c) each set at one of at least 2 chosen temperatures,
e) sampling to get an enumeration sample and determining the growth of the inoculated cells in each vial at minimum 3, preferably at minimum 5, more preferably at from 5 to 9 different incubation times, whereby said determination of the growth of the microbial cells is performed by
(i) fluorescence-staining of the microbial cells,
(ii) enumerating the stained cells by means of a flow cytometer,
f) determining the best of the investigated temperatures and stationary phase time and/or exponential growth time, and optionally redoing steps a) to d) at temperatures close to the best temperature found in the previous run,
wherein at least steps a) to d) are performed using AOC-free and non-AOC-releasing containers, in particular vials, and AOC-free and non-AOC-releasing manipulating means, in particular syringes and filters.

9. The method of claim 1 for determining the microbial growth inhibition ability of a specific compound comprising the steps of
a) providing at least one water sample containing or spiked with at least one known AOC compound at at least one chosen concentration,
b) adding to said water sample the specific compound to be tested in a chosen concentration
c) distributing said sample into multiple identical sets of at least triplicate AOC-free vials, wherein said distribution is performed by means of sterile filtration using a non-AOC-releasing syringe and filter
d) adding a chosen amount (up to 5 x 10⁴ counts.mL⁻¹ final concentration) of an inoculum per vial
e) incubating the at least triplicate inoculated vials of step d) at a selected temperature,
f) sampling to get an enumeration sample and determining the growth of the inoculated cells in each vial after different incubation times until stationary phase is reached and maintained for establishing at least one growth curve, wherein said determination of the growth of the microbial c.ells is performed by
(i) fluorescence-staining of the microbial cells
(ii)enumerating the stained cells by means of a flow cytometer.

10. The method of anyone of the preceding claims, wherein the inoculum is
- one pure culture or
- a combination of pure cultures or
- a natural microbial consortium.

11. The method of anyone of the preceding claims, wherein the inoculum is a natural microbial consortium.

12. The method of anyone of the preceding claims, wherein the incubation is performed at a temperature in the range of 20 to 35°C, preferably 25 to 30°C, in particular in a range of 30 ± 2°C, most preferred at about 30°C.

13. The method of anyone of the preceding claims, wherein at least two different stains are used and at least two independent information sets are obtained.

14. The method of anyone of the preceding claims which is an automated method wherein the inoculated triplicate vials of step c) are given into an incubator, followed by the fully automated steps that an auto-sampler provides an enumeration sample to be stained and enumerated, said enumeration sample being stained, again incubated and then enumerated by flow cytometry.

15. The method of claim 14, wherein the incubation of the stained enumeration sample is performed in a loop incubator.

16. The method of claim 14 or 15, wherein part of the sample after staining and prior to enumeration is automatically separated and diluted by using an automated device.

17. An AOC apparatus for the automated performance of the method of anyone of the preceding claims, comprising a first incubation chamber for at least triplicate vials, preferably for several triplicate vials, an auto-sampling device for automatically withdrawing an enumeration sample to be stained and optionally placing it in a staining container, an automated stain addition means, an automated forwarding means for withdrawing an adequate amount of stained incubated sample and for forwarding it into the flow cytometer, a flow cytometer equipped with at least one, preferably at least two fluorescence detectors for detecting different wavelength and at least one computer for controlling the means and for analyzing the flow cytometry results.

18. The device of claim 17 comprising a second incubator for receiving the staining container containing the stained incubated sample.

19. The device of claim 17, wherein the auto-sampling device is coupled to a flow injection means, said flow injection means comprising a loop incubator and a forwarding means.

20. The device of anyone of claims 17 to 19 comprising a dilution means placed between the stained sample incubation means and the flow cytometer for diluting the stained and incubated sample.
